# EUROPEAN PATENT APPLICATION

(11) **EP 4 653 004 A1**
(43) Date of publication of application: **26.11.2025**
(21) Application number: 24744697.4
(22) Date of filing: 18.01.2024
(51) Int. Cl.: A61K 35/12, A61P 25/28, B01D 61/14, B01D 61/16, B01D 61/22, B01D 63/02, B01J 41/10, B01J 41/13, B01J 41/14, B01J 41/16, B01J 41/18, B01J 41/20

(54) **PHARMACEUTICAL COMPOSITION FOR TRANSNASAL ADMINISTRATION**

(30) Priority: 18.01.2023 JP 2023006176
(71) Applicant: Daicel Corporation, Osaka-shi, Osaka 530-0011 (JP); National University Corporation Hokkaido University, Hokkaido 060-0808 (JP)
(72) Inventor: UCHIMURA, Seiichi, Tokyo 108-8230 (JP); NAKATSUKA, Shuji, Tokyo 108-8230 (JP); KAWABORI, Masahito, Sapporo-shi, Hokkaido 060-0808 (JP)
(74) Representative: Grünecker Patent- und Rechtsanwälte PartG mbB
(86) International application number: PCT/JP2024/001275
(87) International publication number: WO 2024/154776

(57) **Abstract**

The purpose of the present disclosure is to provide a pharmaceutical composition having an excellent therapeutic effect for the treatment of a central nervous system disease. The pharmaceutical composition for transnasal administration contains a purified extracellular vesicle product and is used for treating a central nervous system disease. The purified extracellular vesicle product is obtained by a method for producing a purified extracellular vesicle product, with the method including: (i) purifying a liquid to be treated and containing extracellular vesicles and an impurity by bringing the liquid into contact with an exclusion and anion exchange carrier to obtain a treated liquid containing the extracellular vesicles; and (ii) subjecting the treated liquid to membrane filtration to obtain a concentrate of the extracellular vesicles. The resulting pharmaceutical composition for transnasal administration exhibits an excellent therapeutic effect for the treatment of a central nervous system disease.

## Description

### Technical Field

The present disclosure relates to a pharmaceutical composition for transnasal administration, the pharmaceutical composition being used for treating a central nervous system disease.

### Background Art

When a brain is severely damaged by cardiac arrest, myocardial infarction, asphyxia, various types of shock, or the like, the brain becomes impaired and may suffer from hypoxic encephalopathy. In treating hypoxic encephalopathy, it is important not only to maintain blood pressure but also to maintain blood flow to organs and peripheral tissues throughout a body. Hypoxic encephalopathy has low survival rates of 14% in Japan and from 20% to 30% in the United States, and low rehabilitation rates of 7% in Japan and 9% in the United States (Non-Patent Literature 1 and 2). At present, the only treatment available for hypoxic encephalopathy is therapeutic hypothermia to prevent the progression of brain damage.

An exosome is a type of extracellular vesicle that is a granular substance having a diameter of from 50 to 200 nm and is secreted from a cell. The surface of an exosome contains lipids and proteins derived from the cell membrane, and the interior of an exosome contains intracellular substances such as nucleic acids (miRNA, mRNA, DNA, etc.) and proteins, and exosomes are considered to play a role in information transfer between cells.

Exosomes have been reported to be involved in various diseases such as cancer and neurodegenerative diseases. The types and amounts of functional molecules encapsulated in an exosome or present in an exosome membrane vary depending on the disease, and therefore the use of an exosome for disease detection or prognosis prediction, or as a therapeutic target is expected.

The use of an exosome as a natural drug delivery system (DDS), which differs from an artificial substance such as a liposome, is also expected, and an attempt is being made to deliver an siRNA, an miRNA, a low molecular weight compound or the like to a target cell. Various cell adhesion molecules and carbohydrate chains are expressed on the membrane surface of an exosome, and the expression pattern has been reported to determine a cell type for which an exosome has affinity. A novel DDS is also being developed by modifying and applying the characteristics of an exosome.

While much research and development has been conducted on exosomes and extracellular vesicles, standard and efficient techniques for their production and separation are still under development. In a biological sample containing extracellular vesicles, many proteins and cells having physical and chemical properties similar to those of the extracellular vesicles coexist, and thus the separation of the extracellular vesicles is inherently complex.

The main separation methods currently used are methods that use differences in the density and size of extracellular vesicles and differences in specific surface markers, and specific examples thereof include ultracentrifugation, precipitation, filtration, size exclusion chromatography, and immunoaffinity methods.

The ultracentrifugation method is a separation method that uses differences in density and size among cells, extracellular vesicles (EVs), and proteins, and is used as a typical method for separating extracellular vesicles (Non-Patent Literature 3). However, drawbacks of the ultracentrifugation method include the need for a long amount of time to collect the extracellular vesicles, poor throughput, and co-precipitation of protein aggregates.

The precipitation method was developed as a method that does not use ultracentrifugation, which has problems in terms of the collection time and throughput, and extracellular vesicle/exosome isolation kits based on the precipitation method are now being sold.

The filtration method is a method for separating relatively large vesicle components such as cells and extracellular vesicles in a biological sample using a commercially available membrane filter (for example, a filter made of polyvinylidene difluoride (PVDF) or polycarbonate and having a pore diameter of approximately from 50 to 450 nm), and usually, filtration needs to be further combined with ultracentrifugation for separating extracellular vesicles or exosomes from protein.

### (Non-Patent Literature 4, 5, and 6)

The size exclusion chromatography method has been used to separate extracellular vesicles or exosomes from protein aggregates. Usually, centrifugation or filtration is initially performed to remove cells and extracellular vesicles. The extracellular vesicles such as exosomes then are separated using a commercially available size exclusion column. (Non-Patent Literature 4, 7, 8, 9, and 10)

The immunoaffinity separation method is a method that uses differences in specific surface markers to separate extracellular vesicles such as exosomes. However, the immunoaffinity separation method is generally not suitable for separating extracellular vesicles or exosomes from a large amount of biological sample. Non-Patent Literature 11

These known separation methods require specialized experimental devices or experimental reagents, or multi-step processes, and the reality is that analyzing extracellular vesicles such as exosomes as a routine diagnostic tool in clinical settings is associated with many difficulties (Non-Patent Literature 12).

### Citation List

### Non-Patent Literature

Non-Patent Literature 1: Neurotherapeutics, 2020 Apr; 17 (2), pp. 539-562.
Non-Patent Literature 2: The Lancet Journal, Vol. 369, Issue 9577, pp. 1925, 2007
Non-Patent Literature 3: Biol. Chem. 394, No. 10 (2013), pp. 1253 to 1262, DOI: 10.1515/hsz-2013-0141.
Non-Patent Literature 4: Kidney Int. 82, No. 9 (2012): pp. 1024-1032. DOI: 10.1038/ki.2012.256.
Non-Patent Literature 5: Nat. Biotechnol. 32, No. 5, pp. 490-495 (2014), DOI: 10.1038/nbt.2886
Non Patent Literature 6: Cancer 119 (2013): pp. 1159-1167, DOI: 10.1002/cncr.27895.
Non-Patent Literature 7: Nanomedicine 11, No. 4 (2015): pp, 879-883, doi.org/10.1016/j.nano.2015.01.003.
Non Patent Literature 8: J. Immunol. Methods 411 (2014): pp. 55-65, DOI: 10.1016/j.jim.2014.06.007.
Non-Patent Literature 9: PLoS One 10 (2015): e0145686
Non-Patent Literature 10: J. Extracell. Vesicles 3 (2014): 23430, DOI: 10.3402/jev.v3.23430.
Non-Patent Literature 11: Biol. Chem. 394, No. 10 (2013): pp. 1253-1262, DOI: 10.1515/hsz-2013-0141.
Non-Patent Literature 12: CRDS Center for Research and Development Strategy, Japan Science and Technology Agency, Overview Report, Life Science and Clinical Medicine Field (2021), pp. 499-519

### Summary of Invention

### Technical Problem

There is still room for improvement in therapeutic effects on central nervous system diseases such as hypoxic encephalopathy, and creation of a novel pharmaceutical composition with an improved effectiveness on these types of central nervous system diseases is desired. Although research has been conducted on the relationship between an exosome and a disease, the main focus has been on improving the efficiency of methods for separating an extracellular vesicle such as an exosome. Therefore, a method for producing a purified product that is highly effective in a specific therapeutic application has not been examined.

Thus, an object of the present disclosure is to provide a pharmaceutical composition having an excellent therapeutic effect for treating a central nervous system disease.

### Solution to Problem

As a result of intensive studies, the present inventors found that an excellent therapeutic effect for treating a central nervous system disease can be achieved by preparing, as a composition for transnasal administration, a purified extracellular vesicle product produced by a unique method. The present disclosure was completed through further examinations based on this finding.

The present disclosure provides the following aspects.

Aspect 1. A pharmaceutical composition for transnasal administration, the pharmaceutical composition including a purified extracellular vesicle product and being used for treating a central nervous system disease,
the purified extracellular vesicle product being obtained by a method for producing a purified extracellular vesicle product, the production method including the following (i) purification and (ii) membrane filtration:
(i) purifying a liquid to be treated, the liquid containing extracellular vesicles and an impurity, by bringing the liquid into contact with an exclusion and anion exchange carrier to obtain a treated liquid containing the extracellular vesicles; and
(ii) subjecting the treated liquid to membrane filtration to obtain a concentrate of the extracellular vesicles.

Aspect 2. The pharmaceutical composition for transnasal administration according to aspect 1, in which the central nervous system disease is hypoxic encephalopathy.

Aspect 3. The pharmaceutical composition for transnasal administration according to aspect 2, in which the hypoxic encephalopathy is global cerebral ischemia.

Aspect 4. The pharmaceutical composition for transnasal administration according to aspect 2 or 3, the pharmaceutical composition containing the extracellular vesicles at 1 × 10³ extracellular vesicles/µL or more.

Aspect 5. The pharmaceutical composition for transnasal administration according to any one of aspects 1 to 4, wherein the liquid to be treated is a culture supernatant of cells or a tissue thereof that secretes the extracellular vesicles.

Aspect 6. The pharmaceutical composition for transnasal administration according to any one of aspects 1 to 5, in which the exclusion and anion exchange carrier is a porous carrier having internally positively charged pores.

Aspect 7. The pharmaceutical composition for transnasal administration according to any one of aspects 1 to 6, in which a membrane used for the membrane filtration has a molecular weight cutoff of from 100000 to 1000000.

Aspect 8. The pharmaceutical composition for transnasal administration according to any one of aspects 1 to 7, wherein the membrane used for the membrane filtration has a γ-globulin permeability of from 10% to 80%.

Aspect 9. The pharmaceutical composition for transnasal administration according to any one of aspects 1 to 8, wherein the membrane used for the membrane filtration has a pure water permeation flow rate at 0.1 MPa of from 500 to 1500 L/m²/Hr.

Aspect 10. The pharmaceutical composition for transnasal administration according to any one of aspects 1 to 9, wherein the membrane used for the membrane filtration is a cellulose-based hydrophilic membrane.

Aspect 11. The pharmaceutical composition for transnasal administration according to any one of aspects 1 to 10, wherein the membrane used for the membrane filtration is a hollow fiber membrane.

Aspect 12. The pharmaceutical composition for transnasal administration according to any one of aspects 1 to 11, wherein the membrane filtration is tangential flow filtration.

Aspect 13. The pharmaceutical composition for transnasal administration according to any one of aspects 1 to 12, wherein a membrane surface velocity in the membrane filtration is from 0.3 m/sec to 2 m/sec.

Aspect 14. The pharmaceutical composition for transnasal administration according to any one of aspects 1 to 13, wherein a concentration of the extracellular vesicles contained in the concentrate of the extracellular vesicles is 10 or more times a concentration of the extracellular vesicles contained in the treated liquid.

Aspect 15. The pharmaceutical composition for transnasal administration according to any one of aspects 1 to 14, in which a concentration of total protein contained in the concentrate of extracellular vesicles is 0.3 or less times a concentration of total protein contained in the liquid to be treated.

Aspect 16. A method for producing a pharmaceutical composition for transnasal administration for treating global cerebral ischemia, the production method including (i) purifying a liquid to be treated, the liquid containing extracellular vesicles and an impurity, by bringing the liquid into contact with an exclusion and anion exchange carrier to obtain a treated liquid containing the extracellular vesicles, and (ii) subjecting the treated liquid to membrane filtration to obtain a concentrate of the extracellular vesicles.

### Advantageous Effects of Invention

According to the present disclosure, a pharmaceutical composition having an excellent therapeutic effect for treating a central nervous system disease is provided. Furthermore, according to the present disclosure, a pharmaceutical composition having an excellent therapeutic effect for treating a central nervous system disease can be quickly produced, and therefore extracellular vesicles can be quickly secured at a concentration and amount necessary for treatment, particularly in clinical settings where timely treatment initiation is critical, such as when dealing with global cerebral ischemia.

### Brief Description of Drawings

FIG. 1 is a schematic view of a device for performing membrane filtration in a method for producing a purified extracellular vesicle product produced in Test Example 1.
FIG. 2 is a schematic view of a device for measuring γ-globulin permeability of a hollow fiber membrane used in membrane filtration.
FIG. 3 presents cross-sections of brain tissues of hypoxic encephalopathy model rats two days after transnasal administration of the pharmaceutical composition for transnasal administration, the pharmaceutical composition thereof containing a purified extracellular vesicle product produced in Test Example 3.
FIG. 4 is a chart showing an effect of improving a survival rate or a proliferation rate of a microglial cell using a purified extracellular vesicle product (purified exosome concentrate A) produced in Test Example 4.
FIG. 5 is charts showing an effect of the purified extracellular vesicle product (purified exosome concentrate A) produced in Test Example 4 on inhibiting the secretion of inflammatory cytokines.

### Description of Embodiments

A pharmaceutical composition according to an embodiment of the present disclosure is a transnasally-administered pharmaceutical composition that contains a purified extracellular vesicle product and is used for treating a central nervous system disease, and the purified extracellular vesicle product is produced by a predetermined production method.

### 1. Predetermined Production Method

The predetermined production method includes the following (i) purification and (ii) membrane filtration. The purified extracellular vesicle product produced by the prescribed production method exhibits an excellent therapeutic effect on the treatment of the central nervous system disease. Furthermore, since the predetermined production method can be used to rapidly prepare a purified extracellular vesicle product at a concentration and amount required for treatment, the production method can also accelerate treatment initiation of a central nervous system disease.
(i) purifying a liquid to be treated, the liquid containing extracellular vesicles and an impurity, by bringing the liquid into contact with an exclusion and anion exchange carrier to obtain a treated liquid containing the extracellular vesicles
(ii) subjecting the treated liquid to membrane filtration to obtain a concentrate of the extracellular vesicles

### 1-1. Liquid to be Treated

The liquid to be treated, which is a material used in the predetermined production method, contains extracellular vesicles and an impurity.

Examples of an extracellular vesicle type include an exosome, a microvesicle, and an apoptotic body, and among these, an exosome is preferred from the viewpoint of further enhancing the therapeutic effect of the pharmaceutical composition according to an embodiment of the present disclosure.

An organism from which the extracellular vesicle is derived is not particularly limited, and examples thereof include a mammal such as a mouse, a cow, and a human.

A tissue, a cell, or a body fluid from which the extracellular vesicles is derived is not particularly limited. Examples of the tissue or the cell include bone marrow, dental pulp, fat, amniotic membrane, umbilical cords, periosteum, perichondrium, and a cell thereof, and examples of the body fluid include blood, umbilical cord blood, plasma, serum, saliva, urine, lacrimal fluid, sweat, breast milk, amniotic fluid, cerebrospinal fluid, bone marrow aspirate, pleural fluid, ascites, synovial fluid, aqueous humor, and vitreous body.

A type of a cell from which the extracellular vesicles are derived is not particularly limited, and may be any of a germ cell, a somatic cell, and a stem cell, but from the viewpoint of further enhancing the therapeutic effect of the pharmaceutical composition according to an embodiment of the present disclosure, a stem cell is preferable. The cell may be either an autologous cell or an allogenic cell for the treatment of a central nervous system disease. Examples of the stem cell include a hematopoietic stem cell, a mesenchymal stem cell, a hepatic stem cell, a pancreatic stem cell, and a skin stem cell, but from the viewpoint of further enhancing the therapeutic effect of the pharmaceutical composition according to an embodiment of the present disclosure, a mesenchymal stem cell is preferable. The origin of the mesenchymal stem cells is not particularly limited, and examples of the origin thereof include bone marrow, dental pulp, blood, fat, amniotic membranes, umbilical cords, umbilical cord blood, periosteum, and perichondrium.

The liquid to be treated is not particularly limited as long as it is a biological sample containing extracellular vesicles and an impurity. The biological sample refers to a culture of a cell or a tissue, or a specimen collected from an organism. From the viewpoint of further enhancing the therapeutic effect of the pharmaceutical composition according to an embodiment of the present disclosure, the biological sample is preferably a culture of a cell or a tissue, and is more preferably a culture of a cell.

The impurity is a component that is mixed together with the extracellular vesicles in the preparation of the biological sample, and examples thereof include a culture medium component, a component generated by culturing, and a component contained in a tissue, a cell, a body fluid, or the like from which the sample was collected. The impurity includes a wide variety of components including a protein.

In the present disclosure, from the viewpoint of further enhancing the therapeutic effect of the pharmaceutical composition, a particularly preferable liquid to be treated is a culture supernatant of a cell or a tissue thereof that secretes the extracellular vesicles, and the most preferable example is a culture supernatant of a mesenchymal stem cell.

### 1-2. Exclusion and Anion Exchange Carrier

The liquid to be treated is brought into contact with an exclusion and anion exchange carrier, and thereby a treated liquid containing extracellular vesicles is obtained.

The exclusion and anion exchange carrier refers to a carrier capable of both size exclusion and anion exchange. To enable size exclusion, a carrier with pores may be used. An exclusion size is, for example, 400 kDa or greater, preferably 500 kDa or greater, more preferably 600 kDa or greater, and still more preferably 650 kDa or greater. Moreover, to enable anion exchange, a positively charged carrier may be used. The exclusion and anion exchange carrier may be a combination of a carrier capable of only size exclusion among size exclusion and anion exchange and a carrier capable of only anion exchange among size exclusion and anion exchange, or may be a carrier capable of both size exclusion and anion exchange.

From the viewpoint of further enhancing the therapeutic effect of the pharmaceutical composition according to an embodiment of the present disclosure, the exclusion and anion exchange carrier is preferably a carrier capable of both size exclusion and anion exchange, and is more preferably a porous carrier having internally positively charged pores. A more preferable example of the porous carrier having internally positively charged pores is a particle in which the pores are internally positively charged and portions other than the pores are not positively charged, and a still more preferable example is a multilayer particle having an inner layer modified with a positively charged ligand and a porous shell layer not modified with the ligand.

A shape of the exclusion and anion exchange carrier is not particularly limited, but a particle shape is preferable. An average particle size of the particle-shaped exclusion and anion exchange carrier is, for example, from 1 to 1000 µm, from 10 to 500 µm, from 20 to 300 µm, or from 20 to 150 µm. The average particle size means the particle size (D50, median diameter) at an integrated value of 50% in a volume-based particle size distribution determined by a laser diffraction/scattering method.

A material of the exclusion and anion exchange carrier is not particularly limited, and examples thereof include a polysaccharide such as cellulose, agarose, starch, amylose, dextran, pullulan, and glucomannan; a polyacrylic acid or a derivative thereof; a synthetic organic polymer such as polyvinyl alcohol, nylon, polysulfone, polyacrylonitrile, polyethylene, polypropylene, and polystyrene; and an inorganic polymer such as glass, porous glass, silica gel, and hydroxyapatite. The material is preferably a polysaccharide, and is more preferably agarose or silica gel.

The purification using the elimination and anion exchange carrier may be performed by a chromatography method or by a batch method.

A concentration of the extracellular vesicles in the treated liquid produced by the purification (process (i)) is, for example, 0.65 or more times, and from the viewpoint of further enhancing the therapeutic effect of the pharmaceutical composition according to an embodiment of the present disclosure, preferably 0.70 or more times, more preferably 0.75 or more times, even more preferably 0.80 or more times, still more preferably 0.9 or more times, and yet even more preferably 0.92 or more times or 0.94 or more times (based on weight) a concentration of the extracellular vesicles in the liquid to be treated, which is the liquid subjected to the purification. An upper limit of the concentration of the extracellular vesicles in the treated liquid is, for example, 1 or less time the concentration of extracellular vesicles in the liquid to be treated.

A concentration of total protein in the treated liquid produced by the purification (process (i)) is, for example, 0.5 or less times, and from the viewpoint of further enhancing the therapeutic effect of the pharmaceutical composition according to an embodiment of the present disclosure, preferably 0.4 or less times, more preferably 0.3 or less times, even more preferably 0.2 or less, and still more preferably 0.15 or less times (based on weight) a concentration of total protein in the liquid to be treated, which is the liquid subjected to the purification. A lower limit of the concentration of total protein in the treated liquid is not particularly limited, and may be, for example, 0.05 or more times, 0.08 or more times, or 0.1 or more times the total protein concentration in the liquid to be treated.

### 1-3. Membrane Filtration

A molecular weight cutoff of a membrane used for membrane filtration is not particularly limited, and is, for example, from 100000 to 1000000. However, from the viewpoint of further enhancing the therapeutic effect of the pharmaceutical composition according to an embodiment of the present disclosure, the molecular weight cutoff is preferably from 150000 to 800000, more preferably 200000 to 600000, further preferably from 230000 to 400000, and still further preferably from 250000 to 350000.

A γ-globulin permeability of the membrane used for membrane filtration is not particularly limited, and is, for example, from 5% to 95%. However, from the viewpoint of further enhancing the therapeutic effect of the pharmaceutical composition according to an embodiment of the present disclosure, the γ-globulin permeability is preferably from 10% to 80%, more preferably from 20% to 70%, even more preferably from 30% to 60%, and still more preferably from 40% to 50%. In a case in which an aqueous γ-globulin solution is filtered through a hollow fiber membrane at a filtration pressure of 0.1 MPa, the γ-globulin permeability is a value (%) derived from the following equation: (γ-globulin concentration in permeate)/(y-globulin concentration in aqueous solution) × 100.

A pure water permeation flow rate at 0.1 MPa of the membrane used for membrane filtration is, for example, from 500 to 1500 L/m²/Hr, and from the viewpoint of further enhancing the therapeutic effect of the pharmaceutical composition according to an embodiment of the present disclosure, the pure water permeation flow rate is preferably from 600 to 1300 L/m²/Hr, and more preferably from 800 to 1100 L/m²/Hr.

A material of the membrane used for membrane filtration is not particularly limited, and examples thereof include a hydrophobic membrane such as a polyethersulfone membrane and a polysulfone membrane, and a hydrophilic membrane such as a cellulose-based membrane, but from the viewpoint of further enhancing the therapeutic effect of the pharmaceutical composition according to an embodiment of the present disclosure, the hydrophilic membrane such as the cellulose-based membrane is preferable. Specific examples of the cellulose-based membrane include a regenerated cellulose membrane and a cellulose ester membrane (specific examples including a cellulose acetate membrane, a cellulose propionate membrane, a cellulose butyrate membrane, and a cellulose benzoate membrane), but from the viewpoint of further enhancing the therapeutic effect of the pharmaceutical composition according to an embodiment of the present disclosure, a cellulose ester membrane is more preferable, and a cellulose acetate membrane is particularly preferable.

A shape of the membrane used for membrane filtration is not particularly limited, but from the viewpoint of further enhancing the therapeutic effect of the pharmaceutical composition according to an embodiment of the present disclosure, a hollow fiber membrane is preferable. An inner diameter of the hollow fiber membrane is, for example, from 0.2 mm to 1.4 mm, and from the viewpoint of further enhancing the therapeutic effect of the pharmaceutical composition according to an embodiment of the present disclosure, the inner diameter of the hollow fiber membrane is preferably from 0.4 mm to 1.2 mm, more preferably from 0.6 mm to 1.0 mm, and even more preferably from 0.7 to 0.9 mm.

As a form of the membrane used for membrane filtration, particularly in a case in which a hollow fiber membrane is used, a bundle of a plurality of (for example, from 10 to 500, from 10 to 200, from 20 to 100, from 50 to 100, from 60 to 90, or from 70 to 80) hollow fiber membranes is preferably used.

The membrane filtration method may be either dead-end filtration or tangential flow (cross-flow) filtration, but from the viewpoint of further enhancing the therapeutic effect of the pharmaceutical composition according to an embodiment of the present disclosure, tangential flow filtration is preferable.

A membrane surface velocity (membrane surface flow rate) in the membrane filtration is, for example, from 0.3 m/sec to 2 m/sec, and from the viewpoint of further enhancing the therapeutic effect of the pharmaceutical composition according to an embodiment of the present disclosure, the membrane surface velocity is preferably from 0.4 m/sec to 1.5 m/sec, more preferably from 0.5 m/sec to 1.3 m/sec, even more preferably from 0.6 m/sec to 1.2 m/sec, and still even more preferably from 0.8 to 1.2 m/sec.

An inlet pressure in the membrane filtration may be, for example, from 0.01 MPa to 0.2 MPa, and from the viewpoint of further enhancing the therapeutic effect of the pharmaceutical composition according to an embodiment of the present disclosure, the inlet pressure is preferably from 0.015 MPa to 0.12MPa, and more preferably from 0.02 MPa to 0.07 MPa.

A concentration of the extracellular vesicles in the extracellular vesicle concentrate produced by the membrane filtration (process (ii)) is, for example, 10 or more times (based on weight), and from the viewpoint of further enhancing the therapeutic effect of the pharmaceutical composition according to an embodiment of the present disclosure, preferably 20 or more times, more preferably 30 or more times, even more preferably 40 or more times, and yet even more preferably 45 or more times, 50 or more times, 80 or more times, or 100 or more times a concentration of the extracellular vesicles in the treated liquid subjected to the membrane filtration. An upper limit of the concentration of the extracellular vesicles in the concentrate is not particularly limited, and may be, for example, 60 or less times or 55 or less times the concentration of the extracellular vesicles in the treated liquid.

A concentration of total protein in the extracellular vesicle concentrate produced by the membrane filtration (process (ii)) is, for example, 0.3 or less times, and from the viewpoint of further enhancing the therapeutic effect of the pharmaceutical composition according to an embodiment of the present disclosure, preferably 0.25 or less times, and more preferably 0.2 or less times (based on weight) a concentration of total protein in the liquid to be treated, which is the liquid subjected to purification (process (i)). A lower limit of the concentration of total protein in the extracellular vesicle concentrate is not particularly limited, and may be, for example, 0.0001 or more times, 0.0005 or more times, 0.001 or more times, 0.05 or more times, 0.1 or more times, or 0.15 or more times the concentration of total protein in the liquid to be treated.

An amount of total protein in the extracellular vesicle concentrate produced by membrane filtration (process (ii)) is, for example, 2 or less times, and from the viewpoint of further enhancing the therapeutic effect of the pharmaceutical composition according to an embodiment of the present disclosure, preferably 1.8 or less times, and more preferably 1.6 or less times (based on weight) an amount of total protein in the treated liquid subjected to the membrane filtration (process (ii)). A lower limit of the concentration of total protein in the extracellular vesicle concentrate is not particularly limited, and may be, for example, 0.4 or more times, 0.7 or more times, or 1 or more times the amount of total protein in the treated liquid.

A volume of the extracellular vesicle concentrate produced through the membrane filtration (process (ii)) is, for example, 10% or less, and from the viewpoint of further enhancing the therapeutic effect of the pharmaceutical composition according to an embodiment of the present disclosure, preferably 5% or less, more preferably 3% or less, and even more preferably 2% or less or 1% or less (based on volume) of a volume of the liquid to be treated, which is the liquid subjected to the purification (process (i)). The volume of the extracellular vesicle concentrate produced through the membrane filtration (process (ii)) may also be 0.005% or more, 0.001%, 0.01% or more, 0.1% or more, 0.5% or more, or 1% or more of the volume of the extracellular vesicle concentrate produced through the membrane filtration (process (ii)).

### 2. Purified Extracellular Vesicle Product

A purified extracellular vesicle product contained in the pharmaceutical composition according to an embodiment of the present disclosure is produced by the above-described predetermined production method. A specific form of the purified extracellular vesicle product includes an extracellular vesicle concentrate produced by the above-described predetermined production method, and a super concentrate or a dried product produced by further removing water from the concentrate. Examples of the method for producing the dried product include freeze-drying, spray-drying, and air-drying.

Examples of an extracellular vesicle type contained in the purified extracellular vesicle product include an exosome, a microvesicle, and an apoptotic body, and a single type thereof may be contained, or a combination of a plurality of types may be contained. Among these extracellular vesicles, an exosome is preferable from the viewpoint of further enhancing the therapeutic effect of the pharmaceutical composition according to an embodiment of the present disclosure.

With respect to a size of the extracellular vesicles contained in the purified extracellular vesicle product, an average particle size of the extracellular vesicles is, for example, from 15 to 250 nm, and from the viewpoint of further enhancing the therapeutic effect of the pharmaceutical composition according to an embodiment of the present disclosure, the average particle size thereof is preferably from 40 to 200 nm, more preferably from 50 to 190 nm or from 55 to 170 nm, even more preferably from 65 to 150 nm, still more preferably from 75 to 130 nm, and yet even more preferably from 80 to 120 nm, from 110 to 120 nm, or from 100 to 120 nm. In the present disclosure, the average particle size refers to a 50% integrated value (D₅₀) determined from a volume-based particle size distribution measured by a laser diffraction/scattering method. A modal diameter of the extracellular vesicles contained in the purified extracellular vesicle product is, for example, from 10 to 200 nm, and from the viewpoint of further enhancing the therapeutic effect of the pharmaceutical composition according to an embodiment of the present disclosure, preferably from 30 to 190 nm or from 30 to 180 nm, more preferably from 50 to 160 nm, even more preferably from 60 to 140 nm, still more preferably from 70 to 120 nm, and yet even more preferably from 76 to 115 nm, from 85 to 116 nm, or from 93 to 115 nm. A particle size distribution width ((D₉₀ - D₁₀/D₅₀) is, for example, from 0.1 to 1.6, and from the viewpoint of further enhancing the therapeutic effect of the pharmaceutical composition according to an embodiment of the present disclosure, preferably from 0.2 to 1.4, more preferably from 0.3 to 1.2, still more preferably from 0.4 to 0.95, yet even more preferably from 0.5 to 0.85, and still even more preferably from 0.6 to 0.9, from 0.65 to 0.85, or from 0.7 to 0.79.

A content of the purified extracellular vesicle product in the pharmaceutical composition according to an embodiment of the present disclosure is not particularly limited as long as the content is acceptable in a composition for transnasal administration and can be administered at a dose that is effective for the treatment of a central nervous system disease. The content of extracellular vesicles is, for example, 1 × 10³ vesicles/µL or more, preferably 1 × 10⁴ vesicles/µL or more, and more preferably 1 × 10⁵ vesicles/µL or more. An upper limit of the content of the purified extracellular vesicle product in the pharmaceutical composition according to an embodiment of the present disclosure is not particularly limited, and may be, for example, 1 × 10¹³ vesicles/µL or less, 1 × 10¹² vesicles/µL or less, 1 × 10¹¹ vesicles/µL or less, or 1 × 10¹⁰ vesicles/µL or less in terms of the amount of extracellular vesicles.

In addition, the content of the purified extracellular vesicle product in the pharmaceutical composition according to an embodiment of the present disclosure may be, for example, from 1 to 100000 µg/mL, from 4 to 10000 µg/mL, or from 8 to 5600 µg/mL in terms of the amount of extracellular vesicles.

### 3. Other Components

The pharmaceutical composition according to an embodiment of the present disclosure contains the above-described purified extracellular vesicle product as an active ingredient, and as appropriate, may or may not contain, as another component, a pharmacologically acceptable base and/or a pharmacologically acceptable additive that is acceptable in a composition for transnasal administration.

Examples of the pharmacologically acceptable base and/or the pharmacologically acceptable additive include an excipient, a thickener, a lubricant, a binder, a disintegrant, a solvent, a solubilizing agent, a suspending agent, an emulsifier, a tonicity agent, a buffer, a soothing agent, a stabilizer, a preservative (an antiseptic), a pH adjuster, a cooling agent, an antioxidant, a wetting agent, an adhesive, and a deodorizer. A single type of these bases and additives may be used alone, or a combination of a plurality of types may be used.

### 4. Formulation Form

The pharmaceutical composition according to an embodiment of the present disclosure may be in the form of either a liquid formulation or a powder formulation that is acceptable for a composition for transnasal administration, and a liquid formulation is preferable. When a liquid formulation is to be prepared, the liquid formulation can be produced by mixing the purified extracellular vesicle product with, as necessary, a solvent, a solubilizing agent, a suspending agent, a tonicity agent, a buffer, and/or a soothing agent, and the like, and dissolving, suspending or emulsifying the purified extracellular vesicle product. Further, a thickener may be added to increase the viscosity and impart a retention property. When a powder formulation is to be prepared, the powder formulation can be produced by mixing the purified extracellular vesicle product with an excipient, a binder, a disintegrant and/or other suitable additives as necessary, and then drying the mixture if necessary.

The pharmaceutical composition according to an embodiment of the present disclosure can be packaged in a container for transnasal administration. As the container for transnasal administration, a commercially available container can be appropriately used.

### 5. Applications

The pharmaceutical composition according to an embodiment of the present disclosure is used for treating a central nervous system disease. The central nervous system disease includes, for example, an ischemic cerebrovascular disease and a neurodegenerative disease. Examples of the ischemic cerebrovascular disease includes cerebral embolism, transient cerebral ischemia, subclavian steal syndrome, Wallenberg syndrome (lateral medullary syndrome), cerebral thrombosis, lacunar infarction, reversible ischemic neurological deficit, cerebral infarction, Moyamoya disease (occlusion in the circle of Willis), hypoxic encephalopathy, venous sinus thrombosis, and postoperative spinal cord ischemia. Examples of the neurodegenerative disease include Alzheimer's disease, Lewy body dementia, Parkinson's disease, and amyotrophic lateral sclerosis. Among these central nervous system diseases, from the viewpoint of further enhancing the therapeutic effect of the pharmaceutical composition according to an embodiment of the present disclosure, the pharmaceutical composition is preferably used to treat an ischemic cerebrovascular disease, and is more preferably used to treat hypoxic encephalopathy. In addition, the production method for obtaining the pharmaceutical composition according to an embodiment of the present disclosure can be used to rapidly prepare a purified extracellular vesicle product at a concentration and amount required for treatment, and therefore the pharmaceutical composition is particularly preferably used to treat, as a central nervous system disease, global cerebral ischemia, which is a highly urgent symptom for which treatment initiation is critical.

The pharmaceutical composition according to an embodiment of the present disclosure is used for transnasal administration. A dosage of the pharmaceutical composition according to an embodiment of the present disclosure for a human can be appropriately determined according to details such as an applicable disease and a symptom level, and the dosage for a human is, for example, 1 × 10⁴ vesicles/kg or more, preferably 1 × 10⁵ vesicles/kg or more, more preferably 1 × 10⁶ vesicles/kg or more, even more preferably 1 × 10⁷ vesicles/kg or more, and still more preferably 5 × 10⁷ vesicles/kg or more, in terms of the amount of the extracellular vesicles. An upper limit of the dosage of the pharmaceutical composition according to an embodiment of the present disclosure may be, for example, 1 × 10¹¹ vesicles/kg or less, 1 × 10¹⁰ vesicles/kg or less, 1 × 10⁹ vesicles/kg or less, or 1 × 10⁸ vesicles/kg or less in terms of the amount of extracellular vesicles.

Each aspect disclosed in the present description can be combined with another feature disclosed in the present description.

### Examples

Hereinafter, the present invention will be more specifically described by examples, but each configuration, a combination of the configurations, and the like in each embodiment are merely examples, and various additions, omissions, substitutions, and other changes may be made as appropriate without departing from the spirit of the present invention. The present disclosure is not limited by the embodiments and is limited only by the claims.

### TEST EXAMPLE 1

### (I) Preparation of Culture Supernatant of Mesenchymal Stem Cells (Liquid to be Treated Containing Extracellular Vesicles and Impurities)

Human-derived mesenchymal stem cells (obtained from 56.4 mL of bone marrow aspirate through mononuclear cell isolation and seeding) were cultured using a cell culture medium (MEMα + 5% allogenic-derived platelet lysates (PL) + 1% antibiotic (penicillin-streptomycin; P/S)). The cells were cultured for 21 days, during which 3.5 L of a culture supernatant was collected.

### (II) Purification (Process (i))

50 mL of a carrier (a porous granular material having internally positively charged pores, an adsorbent capable of adsorbing and retaining negatively charged unwanted substances excluding extracellular vesicles) equilibrated with 50 mM Tris-HCl (pH 7.2) was added to 250 mL (an amount of total protein of 470.5 mg as measured according to the Bradford method) of 3.5 L of the culture supernatant obtained in (I) above. The mixture was then dispensed into 50 mL tubes, and then inverted and mixed at room temperature for 10 minutes using a rotator at a rotational speed of 10 rpm. Subsequently, the mixture was centrifuged at room temperature for 2 minutes at 10000 xg, and the supernatant was filtered using a Stericup 0.22 µm filtration unit (S2GPU11RE, Merck Millipore) to remove the carrier, and a treated liquid containing extracellular vesicles was obtained. An amount of total protein in the resulting treated liquid as measured by the Bradford method was 195.3 mg, and thus the amount of total protein was reduced to 42% of the amount before the purification.

### (III) Membrane Filtration (Process (ii))

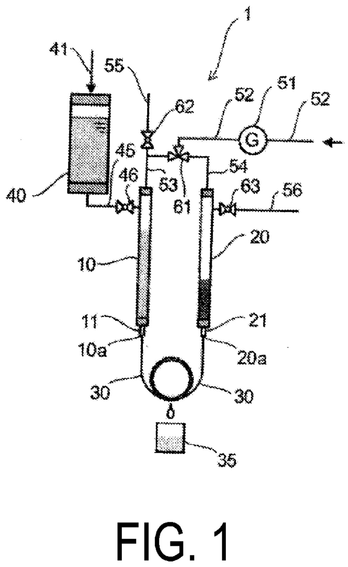

250 mL of the treated liquid produced in (II) above was subjected to membrane filtration using the membrane filtration device 1 shown in FIG. 1.

A membrane filtration device 1 shown in FIG. 1 includes:
· a hollow fiber membrane module 30;
· a first tank 10 and a second tank 20 that are respectively in liquid communication with two ends of the hollow fiber membrane module 30;
· a buffer solution tank 40 that is in openable and closeable liquid communication with the first tank 10 by a buffer solution feed line 45 having an on/off valve 46;
· a first tank gas supply line 53 that is in gas communication with the first tank 10;
· a second tank gas supply line 54 that is in gas communication with the second tank 20;
· a three-way valve 61 configured to allow a gas supply line 52 with a pressure gauge 51 to be in openable and closeable gas communication with the first tank gas supply line 53 or the second tank gas supply line 54;
· a first gas vent line 55 that has an on/off valve 62 and is in openable and closeable gas communication with the first tank 10; and
· a second gas vent line 56 that has an on/off valve 63 and is in openable and closeable gas communication with the second tank 20.

Details of each constituent component of the membrane filtration device 1 are as follows.
· First tank 10 and second tank 20
   Material: Acrylate Resin
   Size: length of 25 cm, inner diameter of 0.25 cm, volume of 120 cm³
· Buffer solution tank 40
   Volume: 1.6 L
· Hollow fiber membrane module 30
   Hollow fiber size: inner diameter of 0.8 mm, outer diameter of 1.3 mm, length of 17 cm
   Material: cellulose acetate (CA)
   Molecular weight cutoff: 300000
   γ-globulin permeability according to the following measurement method: 54%
   Pure water permeation flow rate at 0.1 MPa: 980 L/m²/Hr
   Form: A hollow fiber membrane bundle obtained by bundling 15 cellulose acetate (CA) hollow fiber membranes (available from Daicen Membrane-Systems Ltd.) housed in a cylindrical polycarbonate container, with a membrane area of 0.0062 m².

### Measurement of γ-Globulin Permeability

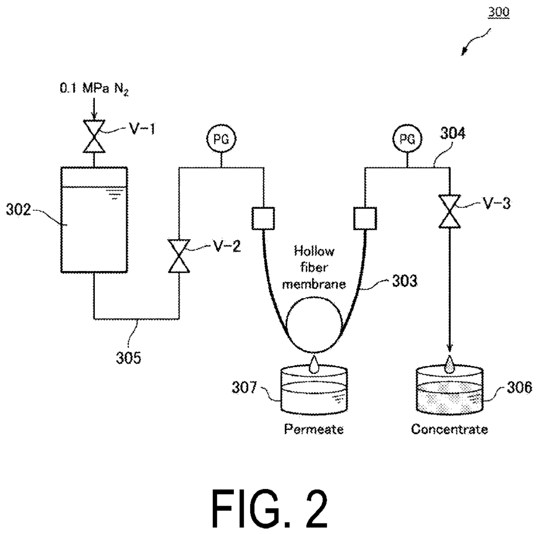

A γ-globulin permeability was measured by the following operation using a γ-globulin permeability measuring apparatus 300 illustrated in FIG. 2.
1) An aqueous γ-globulin solution (y-globulin concentration: 100 ppm) was charged into a tank 302.
2) A hollow fiber membrane 303 was connected to a flow path 305.
3) Nitrogen gas was injected through a valve V-1 to pressurize the tank 302 at 0.1 MPa. The aqueous solution is filtered through the hollow fiber membrane 303 from its inner surface toward its outer surface, a filtrate was discarded into a container 307, and a permeate was recovered into a container 306. Note that an initial permeate (about 100 mL) was discarded, and a permeate discharged thereafter was used as a sample for measuring the permeability.
   After preparing the sample of the permeate by the apparatus 300, the γ-globulin permeability was measured by the following method.
4) A spectrophotometer (available from Shimadzu Corporation, product name "UV2450") is used to measure an absorbance (A1) at 280 nm of an aqueous γ-globulin solution (100 ppm) and an absorbance (A2) at 280 nm of the permeate, and the γ-globulin permeability (%) was calculated by a calculation formula (A2/A1 × 100).

Membrane filtration was performed at approximately 20°C according to the following procedure.
(1) The treated liquid produced in (II) above is placed in the first tank 10.
(2) Nitrogen gas was supplied to the upper space of the first tank 10 at a pressure of 0.04 MPa, and tangential flow filtration was performed by passing a diluted solution of the treated liquid in the first tank 10 through the hollow fiber membrane module 30. At this time, the second tank 20 was opened to the atmosphere by opening the on/off valve 63, and the pressure was zero. A membrane surface linear velocity of flow through the hollow fiber membrane module 30 was 1.0 m/s. The membrane surface linear velocity was calculated from an increasing rate of a concentrate amount in the second tank 20. A permeate of the hollow fiber membrane module 30 was stored in a permeate tank 35, and a concentrate was transferred into the second tank 20. As a result, a diluted solution of the treated liquid in the first tank 10 was passed through the hollow fiber membrane module 30 and subjected to membrane filtration.
(3) When the majority of the treated liquid in the first tank 10 had been subjected to membrane filtration, nitrogen gas was supplied to the second tank 20 at a pressure of 0.04 MPa by switching the three-way valve 61, and at the same time, the pressure in the first tank 10 was released by opening the on/off valve 62. As a result, the concentrate in the second tank 20 was passed through the hollow fiber membrane module 30, and thereby tangential flow filtration in a direction opposite to the direction in (2) above was performed. The permeate was stored in the permeate tank 35, and the concentrate was transferred into the first tank 10. Thereby, the concentrate in the second tank 20 was passed through the hollow fiber membrane module 30 and subjected to membrane filtration.
(4) The concentrate transferred into the first tank 10 was further subjected to tangential flow filtration from the first tank 10 side to the second tank 20 side and tangential flow filtration from the second tank 20 side to the first tank 10 side alternately and repeatedly as indicated in (2) and (3) above until the concentrate reached a liquid amount of 10 mL. To perform backwashing, 90 mL of phosphate buffered saline (PBS) was supplied from a tank (not illustrated) to the hollow fiber membrane module 30, after which the alternating tangential flow filtration and the supply of 90 mL of phosphate buffered saline were repeated two more times. Then, the alternating tangential flow filtration was performed, and thereby 11.2 mL of a purified concentrate (purified extracellular vesicle product) was obtained.

A concentration of total protein in the ultimately obtained purified concentrate of exosomes (purified extracellular vesicle product) was 22.5 mg, and protein was reduced to 4.8% through the purification and membrane filtration.

### (IV) Oxygen-Glucose Deprivation (OGD) Experiment

A cryopreserved neuroblastoma cell line (SH-SY5Y, ATCC) was cultured using an SY5Y dedicated culture medium (eMEM: 45%, F12: 45%, FBS: 10%, P/S: 1%) and then seeded in a 24-well dish (Falcon) at 1.2 x 10⁵/well. Subsequently, in a hypoxic chamber (InvivO2 300), the culture medium was replaced with a glucose-free DMEM culture medium under an atmosphere of 1% oxygen, 5% CO₂, and 94% nitrogen, and oxygen-glucose deprivation (OGD) was applied for 24 hours. After completion of OGD, the cells were returned to a normal incubator (21% oxygen), and the culture medium was replaced with DMEM high glucose (without FBS).

At the time that the culture medium was replaced, the purified concentrate of exosomes (purified extracellular vesicle product) obtained in (III) above was added to the culture medium with the number of exosomes being 1.7 × 10¹⁰ exosomes/10 µL/well. A well to which exosomes were not added was set as a control at the same time. After 72 hours of culturing, the culture medium was aspirated, 500 µL of a detachment solution (TrypLE Select, GIBCO) was added to each well, incubation was performed at 37°C for about 5 minutes, and then neutralization was performed by adding 500 µL of the above culture medium. Pellets obtained by centrifugation were diluted with 1 mL of the culture medium, and the number of cells, survival rate, and the like were measured using the Luna counter (available from Logos Biosystems). The results are shown in Table 1 below.

**[Table 1]**

| | Total Number of Cells (cells/mL) | Survival Rate (%) |
|---|---|---|
| Control | 16550 | 85 |
| Extracellular vesicle quantity of 1.7 x 10¹⁰ | 87200 | 90.5 |

As shown in Table 1, the purified extracellular vesicle product obtained through processes (i) and (ii) demonstrated a significantly improved proliferation rate of neuroblasts under oxygen-glucose deprivation (OGD). Further, the purified extracellular vesicle product increased not only the total number of cells, but also the ratio of viable cells (survival rate) in OGD, indicating a remarkably increased number of viable cells.

### TEST EXAMPLE 2

The purified concentrate of exosomes (purified extracellular vesicle product) obtained in Test Example 1 was subjected to particle size analysis using the nanoparticle multi-analyzer qNano (available from Izon Science Limited). An average particle size (50% integrated value (D₅₀) determined from the volume-based particle size distribution measured by the laser diffraction/scattering method) was 84 nm, a modal diameter was 79 nm, a particle size distribution width ((D₉₀ - D₁₀)/D₅₀) was 0.68, and a number of particles was 1.7 x 10¹² particles/mL.

### TEST EXAMPLE 3

### (I) Preparation of Culture Supernatant of Bone Marrow-Derived Mesenchymal Stem Cells (Liquid to be Treated Containing Extracellular Vesicles and Impurities)

Rat bone marrow-derived mesenchymal stem cells (BMSC) were cultured in DMEM + 10% FBS + 1% P/S, and at 80% confluence, the culture medium was changed to one without FBS, and culturing was then carried out for 24 hours. A supernatant was collected and passed through a 0.45 µm filter.

### (II) Purification (Process (i))

30 mL of a carrier (a porous granular material having internally positively charged pores, an adsorbent capable of adsorbing and retaining negatively charged unwanted substances excluding extracellular vesicles) equilibrated with 50 mM Tris-HCl (pH 7.2) was added to 150 mL (an amount of total protein of 350.6 mg as measured according to the Bradford method) of the culture supernatant obtained in (I) above. The mixture was dispensed into 50 mL tubes, and then inverted and mixed at room temperature for 10 minutes using a rotator at a rotational speed of 10 rpm. Subsequently, the mixture was centrifuged at room temperature for 2 minutes at 10000 xg, and the supernatant was filtered using a Stericup 0.22 µm filtration unit (S2GPU11RE, Merck Millipore) to thereby remove the carrier, and a treated liquid containing extracellular vesicles was obtained. An amount of total protein in the resulting treated liquid as measured by the Bradford method was 159.3 mg, and thus the amount of total protein was reduced to 45% of the amount before the purification.

### (III) Membrane Filtration (Process (ii))

The treated liquid obtained in (II) above was subjected to membrane filtration in the same manner as in Test Example 1 (III). An amount of total protein in an ultimately obtained purified concentrate of exosomes (purified extracellular vesicle product) was 4.8 mg, and thus the amount of protein was reduced to 1.4% through the purification and membrane filtration. The obtained purified concentrate of exosomes (purified extracellular vesicle product) was subjected to particle size analysis using the nanoparticle multi-analyzer qNano (available from Izon Science Limited). An average particle size (50% integrated value (D₅₀) determined from the volume-based particle size distribution measured by the laser diffraction/scattering method) was 113 nm, a modal diameter was 105 nm, a particle size distribution width ((D₉₀ - D₁₀)/D₅₀) was 0.70, and a number of particles was 1.6 x 10¹⁰ particles/mL.

### (IV) Animal Experiments

Both vertebral arteries of rats (SD rats, 379 g, 392 g (about 10 weeks old), male) were completely occluded by electrocoagulation and transection. The next day, both internal carotid arteries of the rats were temporarily blocked for 30 minutes using clips, and immediately after the blocking was released, the purified concentrate of exosomes (purified extracellular vesicle product) obtained in (III) was transnasally administered at a dose of 3.2 x 10⁶ exosomes/5 µL/one nasal cavity (6.2 x 10⁶ exosomes/10 µL in terms of both nasal cavities), or PBS was transnasally administered as a control at a dose of 5 µL/one nasal cavity (10 µL in terms of both nasal cavities). This dose corresponds to 8 × 10⁷ exosomes/kg for humans. The conversion to the human dose is reasonably derived on the basis of a brain weight (set as 2 g for a rat and 1300 g for a human) and a body weight of a human (set at 50 kg).

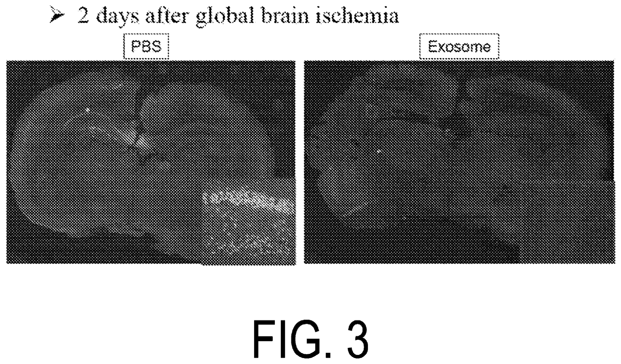

Two days after the administration, paraffin sections of the rat brains were prepared, and apoptotic cells were fluorescently stained with Apoptag (Merck) and observed (with the Keyence BZ-X). The results are shown in FIG. 3. FIG. 3 also shows an enlarged view of tissue in the vicinity of a hippocampus. As shown in FIG. 3, dead cells in the hippocampus were dramatically reduced in an exosome-administered rat (Exosome) compared to a control (PBS).

### Test Example 4

### (1) Preparation of Purified Exosome Concentrate A (Purified Extracellular Vesicle Product)

A treated liquid was prepared in the same manner as in Test Example 1 using a culture supernatant of human amnion-derived mesenchymal stem cells (AMSC).

Approximately 3185 mL of the obtained treated liquid (a mass of total protein of 5159.7 mg as measured by the BCA method, an amount of CD9/CD63 positive exosomes of 512.8 ng as measured by ELISA (in terms of CD9/CD63 fusion standard protein used for a calibration curve)) was filtered using a Stericup 0.22 µm filtration unit (S2GPU11RE, Merck Millipore) and then subjected to membrane filtration in the same manner as in Test Example 1. A hollow fiber membrane module having the following specifications was used.
Hollow fiber size: inner diameter of 0.8 mm, outer diameter of 1.3 mm, length of 17 cm
Material: cellulose acetate (CA)
Molecular weight cutoff: 300000
γ-globulin permeability according to the following measurement method: 54%
Pure water permeation flow rate at 0.1 MPa: 980 L/m²/Hr
Form: A hollow fiber membrane bundle obtained by bundling 125 cellulose acetate (CA) hollow fiber membranes (available from Daicen Membrane-Systems Ltd.) housed in a cylindrical polycarbonate container, with a membrane area of 0.05 m².

An amount of total protein in 26 mL of an ultimately obtained purified concentrate of exosomes (purified extracellular vesicle product) was 35.1 mg, and thus the amount of protein was reduced to 0.7%. In addition, an amount of exosomes was 372.2 ng, and a recovery rate was 73%. Further, when the obtained purified concentrate of exosomes was analyzed using the nanoparticle imaging analyzer VIDEO DROP, an average particle size (50% integrated value (D₅₀) determined from the volume-based particle size distribution measured by the laser diffraction/scattering method) was 187 nm, a modal diameter was 185 nm, a particle size distribution width ((D₉₀ - D₁₀)/D₅₀) was 1.14, and a number of particles was 5.2 x 10¹⁰ particles/mL.

### (2) Preparation of Comparative Purified Exosome Concentrate B by Ultracentrifugation

The culture supernatant of the human amnion-derived mesenchymal stem cells (AMSC) used in (1) above was centrifuged to collect a supernatant twice (conditions of first centrifugation: 2000 g, 10 minutes, 4°C; conditions of first centrifugation: 10000 g, 30 minutes, 4°C). The resulting supernatant was subjected to ultracentrifugation under the following conditions (conditions for ultracentrifugation: 100000 g, 70 minutes, 4°C). The supernatant was discarded, and liquid inside the tubes was removed as much as possible to obtain an exosome precipitate attached to the inner wall of the tubes. The exosome precipitate was suspended in about 200 µL of PBS, and thereby a comparative purified exosome concentrate B was obtained.

An amount of total protein in the ultimately obtained comparative purified exosome concentrate B was 140.4 µg, and when the comparative purified exosome concentrate B was analyzed using the nanoparticle imaging analyzer VIDEO DROP, an average particle size (50% integrated value (D₅₀) determined from the volume-based particle size distribution measured by a laser diffraction/scattering method) was 137 nm, a modal diameter was 135 nm, the particle size distribution width ((D₉₀ - D₁₀)/D₅₀) was 1.08, and a number of particles was 2.3 x 10¹¹ particles/mL.

### (3) Effect of Improving Survival Rate or Proliferation Rate of Microglial Cells

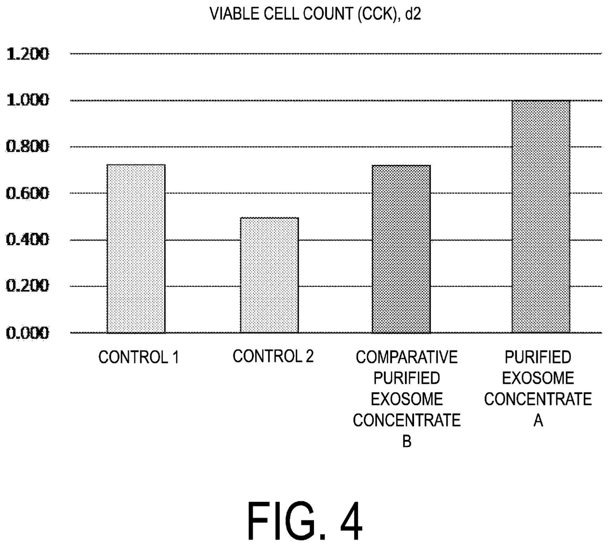

A microglial cell line BV2 (Elabscience) was diluted with a culture medium (MEMα, P/S: 1%) and seeded in a 96-well dish (Falcon) at 2.0 x 10⁴ cells/well. One day later, E. coli 026:B6-derived lipopolysaccharides (L8274, Sigma-Aldrich) were added at an amount of 1000 ng/well, and at the same time, the purified exosome concentrate A obtained in (1) or the comparative purified exosome concentrate B obtained in (2) was added at an amount of 2.8 x 10⁹ exosomes/well, culturing was performed for one day, and then a number of viable cells was counted. CK04 Cell Counting Kit-8 (Dojindo Laboratories) was used to count the number of viable cells, and the absorbance at 450 nm was measured as the number of viable cells. Meanwhile, the same operation was performed with the exception that a PBS solution was added while the lipopolysaccharides and exosomes were not added (Control 1). The same operation was also performed with the exception that exosomes were not added (with lipopolysaccharides added) (Control 2). The results are shown in FIG. 4.

As illustrated in FIG. 4, the survival rate or proliferation rate of the microglial cells can be increased by the addition of the purified exosome concentrate A produced in the above (1), clearly showing a greater effect compared to that of the addition of the comparative purified exosome concentrate B obtained in the above (2).

Microglial cells are activated during a pathological condition, resulting in a drastic change in cell characteristics, accumulate and proliferate in a lesion area, and phagocytose and eliminate damaged nerve cells. It has been confirmed that the addition of the purified exosome concentrate A had an effect of increasing the survival rate or proliferation rate of microglial cells. Therefore, it can be reasonably inferred that a purified extracellular vesicle product produced through (i) purification and (ii) membrane filtration, as with the purified exosome concentrate A, can more effectively promote the phagocytosis and elimination of damaged nerve cells than a purified extracellular vesicle product produced by ultracentrifugation, as with the comparative purified exosome concentrate B.

### (4) Inhibitory Effect on Secretion of Inflammatory Cytokines

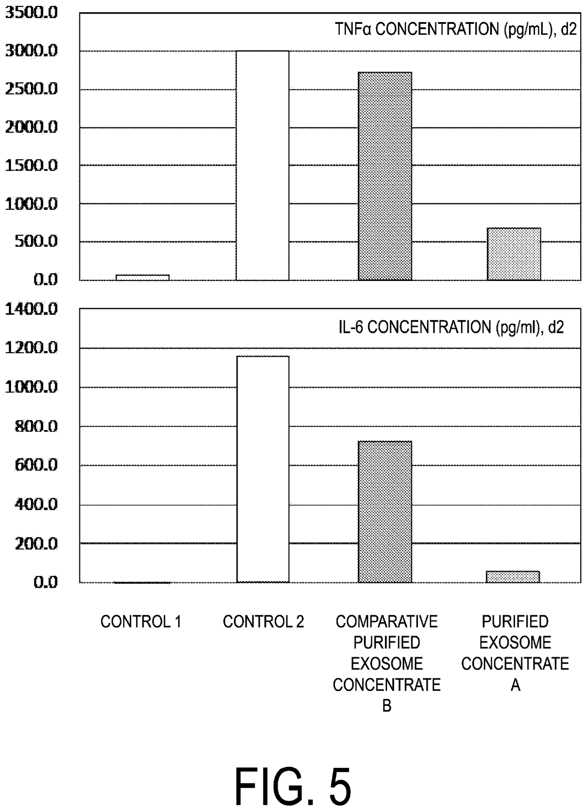

The microglial cell line BV2 (Elabscience) was diluted with a culture medium (MEMα, P/S: 1%) and seeded in a 96-well dish (Falcon) at 2.0 x 10⁴ cells/well. One day later, E. coli 026:B6-derived lipopolysaccharides (L8274, Sigma-Aldrich) were added at an amount of 10 ng/well, and at the same time, the purified exosome concentrate A obtained in (1) or the comparative purified exosome concentrate B obtained in (2) was added at an amount of 2.8 x 10⁹ exosomes/well. After further culturing for 1 day, the culture supernatant was collected, and the amounts of inflammatory cytokines TNFα and IL-6 contained in the supernatant were quantified by ELISA (a Mouse TNF-alpha Quantikine ELISA Kit and a Mouse IL-6 Quantikine ELISA Kit (both available from R&D Systems, Inc.) were used). Meanwhile, the same operation was performed with the exception that a PBS solution was added while the lipopolysaccharides and exosomes were not added (Control 1). The same operation was also performed with the exception that exosomes were not added (with lipopolysaccharides added) (Control 2). The results are shown in FIG. 5.

As illustrated in FIG. 5, it was revealed that the addition of the purified exosome concentrate A produced in (1) above can inhibit a secretion of an inflammatory cytokine, with an effect higher than that of the addition of the comparative purified exosome concentrate B produced in (2) above.

When a brain is damaged, microglial cells are activated and release an inflammatory cytokine such as TNF-α and IL-6, causing an inflammatory response. The inflammatory response is thought to exacerbate a neurological disorder such as multiple sclerosis, a stroke, and Alzheimer's disease, and a psychiatric disorder such as depression, schizophrenia, and autism. It has been confirmed that the addition of the purified exosome concentrate A had an effect of inhibiting the secretion of TNF-α and IL-6 by microglia. Therefore, it can be reasonably inferred that a purified extracellular vesicle product produced through (i) purification and (ii) membrane filtration, as with the purified exosome concentrate A, can better inhibit the secretion of an inflammatory cytokine than a purified extracellular vesicle product produced by ultracentrifugation, as with the comparative purified exosome concentrate B, and thereby the purified extracellular vesicle product produced through (i) purification and (ii) membrane filtration can better inhibit an aggravation of a neurological or psychiatric disorder.

## Claims

1. A pharmaceutical composition for transnasal administration, the pharmaceutical composition comprising a purified extracellular vesicle product and being used for treating a central nervous system disease,
the purified extracellular vesicle product being obtained by a method for producing a purified extracellular vesicle product, the production method comprising (i) purification and (ii) membrane filtration:
(i) purifying a liquid to be treated, the liquid containing extracellular vesicles and an impurity, by bringing the liquid into contact with an exclusion and anion exchange carrier to obtain a treated liquid containing the extracellular vesicles; and
(ii) subjecting the treated liquid to membrane filtration to obtain a concentrate of the extracellular vesicles.

2. The pharmaceutical composition for transnasal administration according to claim 1, wherein the central nervous system disease is hypoxic encephalopathy.

3. The pharmaceutical composition for transnasal administration according to claim 2, wherein the hypoxic encephalopathy is global cerebral ischemia.

4. The pharmaceutical composition for transnasal administration according to claim 2, wherein the pharmaceutical composition comprises the extracellular vesicles at an amount of 1 × 10³ extracellular vesicles /µL or more.

5. The pharmaceutical composition for transnasal administration according to claim 1, wherein the liquid to be treated is a culture supernatant of cells or a tissue thereof that secretes the extracellular vesicles.

6. The pharmaceutical composition for transnasal administration according to claim 1, wherein the exclusion and anion carrier is a porous carrier having internally positively charged pores.

7. The pharmaceutical composition for transnasal administration according to claim 1, wherein a membrane used for the membrane filtration has a molecular weight cutoff of from 100000 to 1000000.

8. The pharmaceutical composition for transnasal administration according to claim 1, wherein a membrane used for the membrane filtration has a γ-globulin permeability of from 10% to 80%.

9. The pharmaceutical composition for transnasal administration according to claim 1, wherein a membrane used for the membrane filtration has a pure water permeation flow rate at 0.1 MPa of from 500 to 1500 L/m²/Hr.

10. The pharmaceutical composition for transnasal administration according to claim 1, wherein a membrane used for the membrane filtration is a cellulose-based hydrophilic membrane.

11. The pharmaceutical composition for transnasal administration according to claim 1, wherein a membrane used for the membrane filtration is a hollow fiber membrane.

12. The pharmaceutical composition for transnasal administration according to claim 1, wherein the membrane filtration is tangential flow filtration.

13. The pharmaceutical composition for transnasal administration according to claim 1, wherein a membrane surface velocity in the membrane filtration is from 0.3 m/sec to 2 m/sec.

14. The pharmaceutical composition for transnasal administration according to claim 1, wherein a concentration of the extracellular vesicles contained in the concentrate of the extracellular vesicles is 10 or more times a concentration of the extracellular vesicles contained in the treated liquid.

15. The pharmaceutical composition for transnasal administration according to claim 1, wherein a concentration of total protein contained in the concentrate of the extracellular vesicles is 0.3 or less times a concentration of total protein contained in the liquid to be treated.

16. A method for producing a pharmaceutical composition for transnasal administration for treating global cerebral ischemia, the production method comprising:
(i) purifying a liquid to be treated, the liquid containing extracellular vesicles and an impurity, by bringing the liquid into contact with an exclusion and anion exchange carrier to obtain a treated liquid containing the extracellular vesicles; and
(ii) subjecting the treated liquid to membrane filtration to obtain a concentrate of the extracellular vesicles.
